# EUROPEAN PATENT SPECIFICATION

(11) **EP 2 173 323 B1**
(45) Date of publication and mention of the grant of the patent: **28.10.2015**
(21) Application number: 08776050.0
(22) Date of filing: 23.07.2008
(51) Int. Cl.: A61K 9/06, A61P 15/00, A61K 31/198, A61K 9/00, A61K 47/38, A61K 47/12, A61K 47/14, A61K 47/10

(54) **STIMULATING GEL**
STIMULIERENDES GEL
GEL STIMULANT

(30) Priority: 01.08.2007 GB 0715018
(43) Date of publication of application: 14.04.2010
(73) Proprietor: LRC Products Limited, Slough, Berkshire SL1 3UH (GB)
(72) Inventor: MORRIS, Lorraine, London EC4V 6BW (GB)
(74) Representative: Carlin, Robert George
(86) International application number: PCT/GB2008/002539
(87) International publication number: WO 2009/016350

(56) References cited:
- EP-A- 1 958 627
- WO-A-2006/103401
- WO-A-2007/044526
- US-A1- 2005 069 597
- US-A1- 2005 244 520
- US-A1- 2005 245 494
- US-A1- 2006 249 162
- US-A1- 2007 048 424
- DATABASE CA CHEMICAL ABSTRACTS SERVICE, COLUMBUS, OHIO, US; XP002519270 retrieved from STN Database accession no. 141:384 274 & BR 2 003 613 A (C. BARROS) 5 August 2003 (2003-08-05)

## Description

The present invention relates to personal lubricants, particularly but not exclusively to personal lubricants which are clitoral stimulating lubricants, and to a method for making them.

Personal lubricants are specialised lubricants which serve to reduce friction with body tissues. In particular, personal lubricants may be used to provide lubrication, or slip, during sexual activity. For example, personal lubricants can be used to increase pleasure or reduce pain during sexual intercourse, and can aid in reducing vaginal dryness. In medicine, personal lubricants may be employed for gynaecological examinations and the like.

A wide variety of personal lubricants are currently available. These lubricants generally function by supplying water on a body surface in a gelled or viscous form, by comprising a water-soluble polymer, such as a water-soluble cellulose derivative, or other water soluble polymers such as polyvinylpyrrolidone, polyvinyl alcohol and the like. In use on a body surface, these gelled or viscous systems retain water on the body surface to which they are applied, and the water provides lubrication. One or more humectants may be added to aid water retention on the body surface, so increasing the level of lubrication (that is, the lubricity of the formulation) provided, and/or increasing the length of time for which lubrication persists (that is, the longevity of lubricity).

In recent years it has become increasingly popular to add various active ingredients to personal lubricant compositions, in order to, for example, enhance pleasurable feelings during sexual activity, and/or to heighten sexual arousal. Personal lubricants comprising such active ingredients are designed to cause physiological or physical changes in the area to which they are applied.

These active ingredient-containing lubricants include clitoral stimulating compositions, which are intended to heighten female sexual excitement, by stimulating blood flow to the clitoris. Clitoral stimulating compositions generally comprise a vasodilator, or vasoactive compound, to increase blood flow. A commonly used vasodilator is L-arginine.

There are a number of personal lubricant compositions on the market which contain L-arginine, usually in conjunction with a coolant which is generally menthol or a menthol derivative, such as a menthoxy compound.

Without wishing to be bound by theory, in the context of personal lubricating compositions, including clitoral stimulating compositions, L-arginine is generally considered to act as a vasodilator to enhance blood flow to the clitoris and surrounding area, and the coolant is thought to provide a cooling, tingling sensation which may provide extra stimulation or sensation. In addition, the coolant is sometimes stated to be a penetration enhancer, enabling the L-arginine to be absorbed more quickly and hence allow the user to be aroused more quickly.

In the context of personal lubricants, coolants are thought to improve desirable sensate properties. This is generally explained by the chemical reaction of coolant compounds on the nerve endings responsible for the sensation of coldness, rather than because of a physical drop in temperature at the body surface (such as would be caused by latent heat of evaporation, for example). In particular, personal lubricant compositions comprising cooling compounds, or coolants, exert cooling and tingling sensations in use, and are considered to have stimulating effects, which are thought to improve and/or aid female and male pleasure.

To date, the coolants that have been used in sexual lubricants include menthol, menthone glycerine acetone and menthyl lactate. Menthol is by far the most commonly used coolant in personal lubricants. However, menthol may be present in combination with other coolants, and these additional coolants are usually menthol- or menthyl-derivatives.

Menthol has a number of negative attributes. Depending upon the menthol concentration, the disadvantages of menthol include: a strong minty smell, which some users find unpleasant; a bitter taste; the potential for causing irritation (including irritation of mucous membranes); the potential for burning at higher concentrations; and high volatility (which has led to eye irritation from some aftershave lotions). The high volatility of menthol also results in a loss of coolant from compositions comprising menthol, so that functionality is lost over time. In addition, high concentrations of menthol, which can occur, for example, as a result of menthol separating out from its carrier, can result in localised burning. Many of these disadvantages, such as menthol's potential to cause irritation and burning, are particularly undesirable in the context of personal lubricant compositions, which are generally applied to particularly sensitive areas of the body. These disadvantages have been found to be shared by many of the commonly used "menthoxy" coolants, which are also commonly used coolants in personal lubricant compositions, often in combination with menthol.

Additional disadvantages of menthol include crystallisation at low temperatures. Crystallisation of menthol in personal lubricant formulations results in clouding of the formulation, and may even lead to a granulated feel in use, which many users find undesirable.

We have found that many of the currently marketed products comprise unacceptably high levels of menthol, which levels may be associated with irritation. Whilst decreasing the menthol concentration can reduce levels of irritation to acceptably low rates, we have found that the very low levels of menthol necessary to minimise irritation also result in reduced menthol functionality. As a consequence, products containing low levels of menthol lack user-perceived benefits such as cooling and tingling sensations and so do not give the desired sensate characteristics in use. Accordingly, the problems associated with menthol cannot be overcome simply by reducing the concentration of menthol used, because products with low levels of menthol have been found to be ineffective.

However, despite its many disadvantages, menthol has long been the coolant of choice in personal lubricant compositions, even in concentrations which may cause irritation. US 2005/069597 discloses a gel with menthol or one of its analogues, together with arginine.

We have now recognised that there is a need for alternative personal lubricant compositions, particularly clitoral stimulating compositions, and have devised compositions which minimize the problems encountered with the prior art compositions.

Subject-matter which is not encompassed by the scope of the claims does not form part of the presently claimed invention.

In its broadest aspect, the present invention provides a personal sexual lubricant composition comprising a vasodilator, and at least one coolant which is not menthol, and which coolant is a cyclic carboxamide, which composition is free from menthol. Any suitable vasodilator can be used. Preferred vasodilators include L-arginine and glyceryl trinitrate.

In one aspect, there is provided a personal sexual lubricant composition comprising a vasodilator and a coolant which is a cyclic carboxamide. The composition is free from menthol. Any suitable cyclic carboxamide may be used. A preferred cyclic carboxamide is Nethyl-p-menthane-3-carboxamide. Any suitable vasodilator can be used, although L-arginine is particularly preferred.

In a preferred aspect, the invention provides a personal sexual lubricant composition comprising a vasodilator; a coolant which is a cyclic carboxamide, and a second coolant.

The composition is free from menthol. Any suitable second coolant can be used.

However, (-)-isopulegol is a particularly preferred second coolant. We have found that the combination of a coolant which is a cyclic carboxamide, such as N-ethyl-p-menthane-3-carboxamide, and isopulegol gives particularly good results, in terms of, for example, the sensations experienced by users.

In another aspect, the invention provides a personal sexual lubricant composition comprising a carrier, a thickening polymer, a vasodilator, a coolant which is a cyclic carboxamide and optionally a second coolant. Preferably, the cyclic carboxamide is N-ethyl-p-menthane-3-carboxamide. Preferably, the second coolant is (-)-isopulegol. Optionally, the composition further comprises a solubiliser, for example to aid solubilisation of the second coolant. When the second coolant is (-)-isopulegol, the composition preferably further comprises a solubiliser. Any suitable solubiliser may be used. Preferred solubilisers include PEG hydrogenated castor oils and polysorbates. PEG 40 hydrogenated castor oil is particularly preferred.

In a preferred embodiment, the invention provides a personal sexual lubricant composition comprising a carrier; a thickening polymer; L-arginine; N-ethyl-p-menthane-3-carboxamide; and isopulegol, which composition is free from menthol. Optionally, the compositions comprise further ingredients such as one or more humectants, one or more preservatives, one or more Ph adjusters; one or more solubilisers; one or more penetration enhancers; and/or one or more additional coolants.

By "penetration enhancer" we mean any compound which aids penetration or absorption of coolants (or other active ingredients) into and through body surfaces, for example, compounds which aid penetration of coolants into and through the skin and mucosal membranes. Preferably, penetration enhancers as used herein aid penetration and/or absorption of coolants which are cyclic carboxamides, such as N-cthyl-p-menthane-3-carboxamide, and/or aid penetration and/or absorption of (-)-isopulegol across body surfaces. Such penetration enhancement may potentiate the action of coolants, or other active ingredients. Any suitable penetration enhancer may be used. Preferred penetration enhancers include ethoxy diglycol, limonene, isopropyl palmitate, sodium lauryl sulphate and L-arginine. Optionally, the penetration enhancer may also act as a vasodilator. For example, and without wishing to be bound by theory, L-arginine is widely considered to act as a vasodilator, although we have found that L-arginine aids penetration of coolants (such as cyclic carboxamides and isopulegol) across body surfaces.

In a highly preferred aspect, the compositions of the invention are aqueous. Aqueous compositions are particularly preferred because they are compatible with all condom types, including natural rubber latex condoms.

In a preferred aspect, the personal lubricant compositions of the invention are gels, particularly lubricious gels, more particularly clitoral stimulating gels. The compositions may be used as topical or personal lubricants.

In a particularly preferred embodiment there is provided a personal sexual lubricant composition comprising a carrier which is water; a thickening polymer which is hydroxyethylcellulose; a humectant which is propylene glycol; a vasodilator/penetration enhancer which is L-arginine; a cyclic carboxamide which is N-ethyl-p-menthane-3-carboxamide; a second coolant which is (-)-isopulegol; a solubiliser which is PEG 40 hydrogenated castor oil; a pH adjuster; and a preservative. Any suitable pH adjuster may be used. Suitably, the pH adjuster is selected for compatibility with the thickening polymer. Preferred pH adjusters include but are not limited ta organic acids and bases such as, for example, lactic acid, citric acid, triethanolamine and aminomethylpropanol. A particularly preferred pH adjuster is lactic acid. The pH adjuster may be added in any suitable amount. Suitably, the pH adjuster is added in an amount suitable for adjusting the pH of the composition to a pH which is suitable for application to the human genital area. A suitable pH is from pH 4.5 to pH 5.5. Any suitable preservative may be used, such as an antifungal preservative and/or an antimicrobial preservative. Suitable preservatives include potassium sorbate, phenoxyethanol, total parabens, benzoic acid and imidazolidinyl urea. A particularly preferred preservative is methylparaben.

The invention also provides a method of making a personal sexual lubricant composition, comprising mixing together the components of the composition as generally described herein.

In another aspect, the invention provides a method of making a personal lubricant composition comprising providing a solvent; a thickening polymer; a vasodilator; a coolant which is a cyclic carboxamide; optionally a second coolant, and mixing the ingredients to form the composition. Preferably, the cyclic carboxamide is N-ethyl-p-menthane-3-carboxamide. Preferably, the second coolant is (-)-isopulegol. Mixing may comprise any suitable mixing method. Suitably, mixing comprises one or more steps of homogenisation.

The personal sexual lubricant compositions of the invention are suitably made by providing a vessel; charging a solvent; adding a vasodilator and optionally a preservative; premixing a thickening polymer and a humectant to form a first premix; adding the first premix to the vessel; separately premixing a coolant which is a cyclic carboxamide and a second coolant and an optional solubiliser to form a second premix; adding the second premix and mixing; and optionally adjusting the pH by adding a pH adjuster.

A method of making a preferred personal sexual lubricant composition comprises providing a vessel; charging water; adding L-arginine and a preservative; premixing hydroxyethylcellulose and propylene glycol (first premix); adding the first premix to the vessel and mixing; separately premixing molten PEG 40 hydrogenated castor oil, (-)-isopulegol and N-ethyl-p-menthane-3-carboxamide (second premix); adding the second premix to the vessel and mixing; and adjusting the pH. The pH may be adjusted to any suitable pH. Suitable pHs include, but are not limited to, pHs suitable for topical application to the human genital area, particularly the female genital area. Suitably, the pH of the composition is adjusted to from about pH 4.5 to about pH 5.5. Mixing may comprise any suitable mixing method, such as, for example, stirring and/or homogenisation. Preferably, mixing comprises one or more steps of homogenisation. It is particularly preferred that the PEG 40 hydrogenated castor oil remains molten throughout the method, and the bulk temperature is preferably chosen accordingly. Suitably, the temperature of the main bulk is maintained at about 30°C prior to addition of the second premix, and preferably the temperature of the main bulk is subsequently maintained at about 30°C.

The compositions provided by the present invention are free, or substantially free, from menthol. Accordingly, the compositions have significant advantages over currently available clitoral stimulating-type lubricants, which almost without exception comprise menthol, because compositions of the invention avoid all the disadvantages inherent in using menthol. The compositions have been found to have the same, or better, user-perceived benefits as known formulations containing a vasodilator (such as L-arginine) and menthol, but have the advantage of being menthol-free. Accordingly, formulations according to the present invention have the significant additional benefits associated with being free from menthol. The compositions provided herein are substantially non-irritating, non-burning and non-cytotoxic. They are also low-odour and less subject to loss of coolant.

N-ethyl-p-menthane-3-carboxamide (WS-3) does not crystallise at low temperatures, and we have found that, in contrast to menthol-containing compositions, N-ethyl-p-menthane-3-carboxamide-containing compositions do not go cloudy after exposure to or storage at low temperatures.

Furthermore, N-ethyl-p-menthane-3-carboxamide has a lower volatility than menthol, so the compositions of the present invention do not suffer from the same loss-of-coolant problems found with menthol-based cooling personal lubricants.

In addition, we have found that N-ethyl-p-menthane-3-carboxamide lacks the volatile side effects of menthol, and provides a smooth, gradual cooling sensation, which has been reported as particularly pleasant by personal lubricant-users.

In preliminary trials, we have found that the compositions have a pleasant lubricant feel, a good duration of sensation and give a pleasant sensation during use. The majority of users reported that the compositions helped them to achieve orgasm. These benefits were retained by compositions even after ageing, in contrast to menthol-containing compositions, which suffer from a loss of menthol with ageing. The compositions are generally very well received by users in comparison to other available L-arginine and menthol-containing personal lubricants.

In the present compositions, menthol is replaced by a coolant which is a cyclic carboxamide, optionally in combination with isopulegol.

Any suitable cyclic carboxamide compound can be used. Cyclic carboxamides are based upon the menthol cyclic structure, but with a C-C link at the 3-position on the ring, in place of a C-O link which is found in the various menthoxy coolants such as menthone glycerine acetal, menthyl lactate and menthoxypropane-1,2-diol.

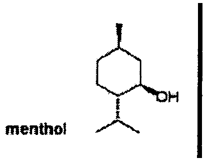

N-ethyl-p-menthane-3-carboxamide (WS-3) is a particularly preferred cyclic carbxamide.

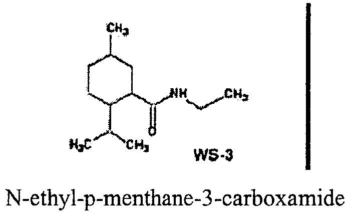

N-ethyl-p-menthane-3-carboxamide has to date primarily been used in oral, medicinal and confectionary products, such as breath-freshening chewing gums. We have found that this compound provides particularly good coolant effects. For example, it lacks the volatile side effects of menthol and provides a gradual, smooth, cooling sensation.

N-ethyl-p-menthane-3-carboxamide has been found to be superior to the coolants that have previously been used in personal lubricants (that is, menthol, menthone glycerine acetal, menthyl lactate and the like). For example, as indicated in Table 1, N-ethyl-p-menthane-3-carboxamide (WS-3) has been found to have a superior relative cooling strength to all the menthoxy coolants.

**Table 1**

| Coolant | Structure | Cooling strength relative to menthol |
|---|---|---|
| Menthoxypropane-1,2-diol | menthoxy | 15.8 - 39.5 |
| Isopulegol | menthoxy | 25 |
| Menthone glycerine acetal | menthoxy | 41 |
| Menthyl lactate | menthoxy | 43 |
| WS-23 | linear carboxamide | 75 |
| Menthol | menthoxy | 100 |
| WS-3 | cyclic carboxamide | 150 |

Preferably, the compositions comprise a coolant which is a cyclic carboxamide in combination with a second coolant. Any suitable second coolant can be used. However, we have found that isopulegol gives a particularly good cooling sensation in use, when used in combination with a cyclic carboxamide such as N-ethyl-p-menthane-3-carboxamide.

By isopulegol, we mean (-)-isopulegol, or 2-isopropenyl-5-methyl-cyclohexanol: 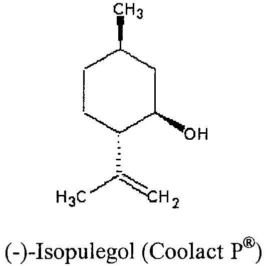 (-)-Isopulegol is sold under the name "Coolact P^{®}" by Takasago International.

Any suitable amount of these coolants can be employed. A preferred amount of coolant is from about 0.01 to about 1.5 % w/w (by weight of the total formulation), more preferably from about 0.1 to about 1.5% w/w. Each coolant may be present in an amount from about 0.01 to about 1.5 % w/w. A more preferred amount of each coolant is from about 0.01 to about 0.5 % w/w. Preferably, the formulation comprises from about 0.016 to about 0.020% w/w N-ethyl-p-menthane-3-carboxamide. Preferably, the formulation comprises from about 0.25 to about 0.3%, for example 0.275%, w/w of (-)-isopulegol. Most preferably, the formulation comprises N-ethyl-p-menthane-3-carboxamide in an amount from about 0.016 to about 0.020% w/w and (-)-isopulegol in an amount about from 0.25 to 0.3%, for example 0.275%, w/w.

The formulations may comprise one or more additional coolants, in addition to (-)-isopulegol and N-ethyl-p-menthane-3-carboxamide. Suitable additional coolants include, but are not limited to, menthyl lactate, menthone glycerine acetal, and menthoxypropane diol. However, menthol is preferably not included in the present formulations, although low levels are not excluded.

The compositions comprise a vasodilator. Any suitable vasodilator may be used. However, preferred vasodilators are L-arginine and glyceryl trinitrate.

Any suitable carrier, or solvent, can be employed. Aqueous compositions are particularly preferred, so water is a preferred carrier. Aqueous compositions are particularly advantageous because they are compatible with all condom types, including natural rubber latex, synthetic polyisoprene and polyurethane condoms. We have found that coolants which are cyclic carboxamides, such as N-ethyl-p-menthane-3-carboxamide, and isopulegol, are compatible with aqueous compositions. Aqueous gel compositions, particularly aqueous lubricious gel compositions, are particularly preferred.

The compositions optionally further comprise one or more additional ingredients, in accordance with conventional formulating practice. For example, the compositions optionally further comprise one or more of a thickening polymer; a humectant; a solubiliser; a penetration enhancer; a preservative; a pH adjuster; and one or more additional coolants.

Any suitable thickening polymer can be used. For example, the thickening polymer may be one or more carbomers; one or more celluloses, such as, for example, hydroxyethylcellulose; or a polyacrylate such as, for example, glyceryl polyacrylate. Glyceryl polyacrylate is commercially available in the form of Lubragel^{™} compositions such as, for example, Lubragel II XD^{™} (commercially available from United-Guardian, Inc). We have found that Lubragel II XD^{™} provides particularly good lubricity, particularly after dilution. Accordingly, of the Lubragels^{™} available, Lubragel II XD^{™} is particularly preferred. Lubragel II XD^{™} has the following composition:

| | |
|---|---|
| Water | 40 - 50% |
| Glycerin | 40 - 50% |
| Glyceryl polyacrylate | 1 - 5% |
| Methylparaben | 0.045- 0.055% |
| Propylparaben | 0.027 - 0.033% |

Accordingly, the thickening polymer may be glyceryl polyacrylate in the form of a Lubragel^{™} composition, preferably Lubragel II XD^{™}.

The formulation may optionally comprise a mixture of two or more thickeners.

A particularly preferred thickening polymer is hydroxyethylcellulose. A suitable hydroxyethylcellulose is Natrosol^{™} 250 HHX (commercially available from Hercules, Inc).

The thickening polymer may be included in the composition in any suitable amount. Preferred amounts of thickening polymer include amounts from about 0.5% w/w to about 3.0% w/w (by weight of the total composition). A more preferred amount of thickener is an amount from about 1.0% w/w to about 2.0% w/w. A most preferred amount of thickener is about 1.5% w/w. In a particularly preferred embodiment, the thickener is hydroxyethylcellulose in an amount of about 1.5% w/w by weight of the composition.

Any suitable humectant can be used. For example, suitable humectants include, but are not limited to, glycerine; polyols such as, for example, sorbitol and mannitol; polyethylene glycols; and propylene glycols. A preferred humectant is propylene glycol. Any suitable amount of humectant can be used. The humectant is typically present in an amount from about 5 to about 50% w/w (by weight of the total formulation). Preferably, the humectant is present in an amount from about 10 to about 40% w/w. More preferably, about 25% w/w humectant is used. In a particularly preferred embodiment, the composition comprises 25% w/w propylene glycol.

Optionally the compositions further comprise a solubiliser. Any suitable solubiliser may be used. For example, a solubiliser may aid in solubilisation of (-)-isopulegol; when (-)-isopulegol is present, the compositions suitably further comprise a solubiliser for (-)-isopulegol. Preferred solubilisers include polyethylene glycol (PEG) hydrogenated castor oils and polysorbates. PEG-hydrogenated castor oils are particularly preferred. In a particularly preferred embodiment, the formulation comprises PEG 40 hydrogenated castor oil.

Optionally, the compositions further comprise a penetration enhancer. The penetration enhancer can be any compound which aids penetration or absorption of the cyclic carboxamide and/or of isopulegol into and through body surfaces, for example, compounds which aid penetration into and through the skin and mucosal membranes. Any suitable penetration enhancer may be used. Preferred penetration enhancers include ethoxy diglycol, limonene; isopropyl palmitate; and sodium lauryl sulphate. In addition, without wishing to be bound by theory, we have found that L-arginine, which is generally considered to be a vasodilator, also functions as a penetration enhacer for coolants such as cyclic carboxamides and isopulegol. Accordingly, because of its dual role as a vasodilator and a penetration enhancer, compsositions comprising L-arginine are particularly preferred. Optionally, the formulation may comprise one or more additional penetration enhancers, in addition to L-arginine.

The compositions optionally further comprise a pH adjuster. Any suitable pH adjuster may be used. Suitable pH adjustors are typically selected in view of the thickening polymer used. Preferred pH adjusters include organic acids and bases. Suitable organic acids include, but are not limited to, lactic acid and citric acid. Suitable organic bases include, but are not limited to, bases such as triethanolamine and aminomethylpropanol. Lactic acid is a particularly preferred pH adjuster. For example, lactic acid is commercially available from Purac Biochem (UK) Ltd, under the trade name Purac^{™} PH90.

The pH adjuster may be included in any suitable amount. Preferably, the pH adjuster is included in an amount sufficient to adjust the pH of the composition to a pH which is suitable for topical application to the human genital area, particularly the female genital area. Suitably, the pH adjuster is sufficient to adjust the pH of the composition to from about pH 4.5 to about pH 5.5.

Optionally, the formulation further comprises a preservative, such as an antimicrobial preservative. Any suitable preservative may be used. However, preferred preservatives include potassium sorbate, phenoxyethanol, total parabens, benzoic acid, imidazolidinyl urea and the like. Optionally, mixtures of two or more preservatives can be used. A particularly preferred preservative is methylparaben.

The following table indicates preferred amounts of ingredients which may suitably be employed in the present invention.

**Table 2**

| Ingredient | %w/w |
|---|---|
| Humectant/moisturiser | 5.0 - 50.0 |
| Thickening polymer | 0.5 - 3.0 |
| Vasodilator | 1.0 - 5.0 |
| Cooling agents | 0.1 - 1.5 |
| Penetration Enhancer | 0 - 5.0 |
| pH adjuster | As required, to give pH 4.5 - 5.5 |
| Preservative | 0.1 - 0.4 |
| Carrier (water) | to 100 |

The compositions may be manufactured by any suitable process. Generally, the compositions will be manufactured by mixing together the components of the composition. Any suitable mixing process may be employed, and such mixing processes will be known in the art. Preferably, however, mixing comprises one or more steps of homogenisation.

More particularly, the method comprises mixing a carrier, or solvent, a vasodilator, a thickening polymer, a humectant, a coolant which is a cyclic carboxamide, and optionally a second coolant which is preferably isopulegol, Optionally, the method further comprises adding a solubilser. Optionally, the method further comprises adding a preservative. The method optionally further comprises adjusting the pH by addition of a pH adjuster. Preferably, the pH is adjusted to a pH which is suitable for topical application to the human genital area.

Preferably, the process generally comprises the following steps:
1) charging water into a vessel;
2) adding vasodilator and optional preservative to the water and stirring;
3) providing a pre-blend of thickening polymer and humectant and adding the pre-blend to the water, vasodilator and optional preservative in the vessel;
4) in a separate vessel, pre-mixing the optional solubiliser and coolants (-)-isopulegol and WS-3;
5) adding the pre-mix from step (5) to the main vessel and stirring; and
6) optionally adding pH adjustor to adjust the pH of the formulation to the required level, and stirring until the formulation becomes homogenous.

Preferably, the method comprises a step of adding a solubiliser (in step 4). A preferred solubiliser is PEG 40 hydrogenated castor oil, which serves to solubilise (-)-isopulegol. Preferably, PEG 40 hydrogenated castor oil is added as molten PEG 40 castor oil and, more preferably, it remains molten throughout the manufacturing process. Preferably, therefore, the temperatures at which each step is carried out are selected accordingly. For example, the optional solubiliser is preferably warmed prior to mixing with the coolants, for example, the optional solibiliser may suitably be warmed to a temperature of about 40°C or below prior to the addition of the coolants. Suitably, the solubiliser (such as PEG 40 hydrogenated castor oil) may be warmed by placing it in a warm room, although any method of warming may be used. Once the solubiliser and coolants have been mixed, the temperature is preferably maintained at from about 30°C to about 35°C.

Suitably, the bulk mix is cooled to a temperature of from about 25 to about 30°C before the premix is added. Preferably, the temperature of the bulk mix is maintained at about 30°C or below once the premix comprising the coolants has been added. More preferably, the bulk mix is maintained at a temperature of about 30°C or below until the product is filled. Preferably, the vessel is also kept closed at all times, apart from opening for addition of materials or for sampling, until the product is filled. Keeping the vessel closed aids in preventing loss of coolant by evaporation.

Preferably, the optional pH adjuster is added to adjust the pH to within a pH range suitable for applying to the female genital area. For example, the pH is suitably adjusted to from about pH 4.5 to about pH 5.5.

Optionally, the method further comprises a step of adding a penetration enhancer.

The following Example illustrates preferred embodiments of the invention.

### Example 1

| | | % w/w |
|---|---|---|
| Humectant/moisturiser | Propylene glycol | 25.000 |
| Thickening polymer | Hydroxyethylcellulose | 1.5000 |
| Vasodilator | L-arginine | 2.000 |
| Cooling agent | (-)-isopulegol | 0.275 |
| Cooling agent | WS-3 | 0.016 - 0.020 |
| Solubiliser | PEG 40 hydrogenated castor oil | 0.500 |
| pH adjuster | Lactic acid (90%) | 1.550 |
| Preservative | Methylparaben | 0.180 |
| Carrier | Water | to 100 |

The composition was made by the following method.

202.5 litres of water was charged into a vessel, with stirring. L-arginine and methylparaben were added with stirring, and homogenised until dissolved. In the main vessel, hydroxyethylcellulose (Natrosol^{™} 250 HHX) and propylene glycol were pre-blended, mixed until homogenous, then poured carefully into a vortex with the homogeniser switched to medium/high speed.

Homogenisation was carried out until Natrosol^{™} 250 HHX was homogenous. The product was checked to ensure no lumps were present.

Pre-melted PEG 40 hydrogenated castor oil was added to a separate premix vessel, which was placed in a warm room, and maintained at below 40°C until use. The temperature of the PEG 40 hydrogenated castor oil was checked, and then (-)-isopulegol and WS-3 were added and mixed until homogenous. The temperature of this premix was maintained at from 30 to 35°C. The PEG 40 hydrogenated castor oil was kept molten throughout the mixing process. Once Natrosol^{™} 250 HHX had fully dissolved in the main vessel, the homogeniser was switched off, and the stirrer was switched on to medium speed. The main bulk was chilled to from 25 to 30°C, and then the premix was added and stirred continuously until the solution was homogenous. After addition of the premix, the bulk temperature was maintained at 30°C or below until the composition was filled into suitable vessels. In addition, the main vessel was kept closed subsequent to addition of the premix, apart from opening for the addition of materials or for sampling.

Lactic acid was added and the main bulk was stirred until homogenous, and checked to make sure no lumps were present. The pH was adjusted to from pH 4.5 to pH 5.5. The final product was filled into screw-top containers.

## Claims

1. A personal sexual lubricant composition comprising a vasodilator and at least one coolant which is a cyclic carboxamide, wherein the composition is free from menthol.

2. A personal sexual lubricant composition according to claim 1 wherein the cyclic carboxamide is N-ethyl-p-menthane-3-carboxamide.

3. A personal sexual lubricant composition according to claim 1 or 2 further comprising a second coolant, wherein the second coolant is (-)isopulegol, menthyl lactate, menthone glycerine acetal or menthoxypropane diol.

4. A personal sexual lubricant composition according to claim 3 wherein the second coolant is (-) isopulegol.

5. A personal sexual lubricant composition according to any one of claims 1 to 4 further comprising a carrier; a thickening polymer; and optionally a solubiliser.

6. A personal sexual lubricant composition according to claim 5 comprising a second coolant which is (-) isopulegol and a solubiliser which aids solubilisation of (-) isopulegol.

7. A personal sexual lubricant composition according to claim 6 wherein the solubiliser is selected from PEG hydrogenated castor oils and polysorbates, optionally wherein the solubiliser is PEG 40 hydrogenated castor oil.

8. A personal sexual lubricant composition according to any preceding claim wherein the vasodilator is selected from L-arginine and glyceryl trinitrate.

9. A personal sexual lubricant composition according to claim 5 wherein the vasodilator comprises L-arginine; the cyclic carboxamide is N-ethyl-p-menthane-3-carboxamide; the second coolant is (-) isopulegol, which composition optionally comprises one or more further ingredients selected from at least one humectant; at least one preservative; at least one pH adjuster; at least one solubiliser; at least one penetration enhancer; and at least one additional coolant.

10. A personal sexual lubricant composition according to 9 comprising a carrier comprising water; a thickening polymer which is hydroxyethylcellulose; a humectant comprising propylene glycol; a solubiliser which is PEG 40 hydrogenated castor oil; a pH adjuster and a preservative.

11. A personal sexual lubricant composition according to any preceding claim which is formulated as a gel.

12. A method of making a personal sexual lubricant composition comprising providing a carrier; a thickening polymer; a vasodilator; a coolant which is a cyclic carboxamide optionally which is N-ethyl-p-menthane-3-carboxamide; and optionally a second coolant, and mixing the ingredients to form the composition, wherein the composition is free of menthol.

13. A method according to claim 12 wherein the second coolant is (-) isopulegol.

14. A method according to claim 12 or 13 comprising providing a vessel; charging a carrier; adding a vasodilator and optionally a preservative; premixing a thickening polymer and a humectant to form a first premix; adding the first premix to the vessel; separately premixing a coolant which is a cyclic carboxamide and a second coolant and optionally a stabiliser to form a second premix; adding the second premix to the vessel; mixing; and optionally adjusting the pH by adding one or more pH adjusters.

## Patentansprüche

1. Persönliche Sexualgleitmittelzusammensetzung, umfassend einen Vasodilator und wenigstens ein kühlendes Mittel, das ein cyclisches Carboxamid ist, wobei die Zusammensetzung frei von Menthol ist.

2. Persönliche Sexualgleitmittelzusammensetzung gemäß Anspruch 1, wobei das cyclische Carboxamid N-Ethyl-p-menthan-3-carboxamid ist.

3. Persönliche Sexualgleitmittelzusammensetzung gemäß Anspruch 1 oder 2, ferner umfassend ein zweites kühlendes Mittel, wobei das zweite kühlende Mittel (-)-Isopulegol, Menthyllactat, Menthonglycerinacetal oder Menthoxypropandiol ist.

4. Persönliche Sexualgleitmittelzusammensetzung gemäß Anspruch 3, wobei das zweite kühlende Mittel (-)-Isopulegol ist.

5. Persönliche Sexualgleitmittelzusammensetzung gemäß einem der Ansprüche 1 bis 4, ferner umfassend einen Träger; ein verdickendes Polymer; und gegebenenfalls einen Solubilisator.

6. Persönliche Sexualgleitmittelzusammensetzung gemäß Anspruch 5, umfassend ein zweites kühlendes Mittel, das (-)-Isopulegol ist, und einen Solubilisator, der die Solubilisierung von (-)-Isopulegol unterstützt.

7. Persönliche Sexualgleitmittelzusammensetzung gemäß Anspruch 6, wobei der Solubilisator ausgewählt ist aus PEG-hydrierten Rizinusölen und Polysorbaten, gegebenenfalls wobei der Solubilisator PEG-40-hydriertes Rizinusöl ist.

8. Persönliche Sexualgleitmittelzusammensetzung gemäß einem der vorstehenden Ansprüche, wobei der Vasodilator ausgewählt ist aus L-Arginin und Glyceryltrinitrat.

9. Persönliche Sexualgleitmittelzusammensetzung gemäß Anspruch 5, wobei der Vasodilator L-Arginin umfasst; das cyclische Carboxamid N-Ethyl-p-menthan-3-carboxamid ist; das zweite kühlende Mittel (-)-Isopulegol ist, welche Zusammensetzung gegebenenfalls einen oder mehrere weitere Inhaltsstoffe ausgewählt aus wenigstens einem Feuchthaltemittel; wenigstens einem Konservierungsmittel; wenigstens einem pH-Wert-Regulator; wenigstens einem Solubilisator; wenigstens einem Penetrationsverstärker; und wenigstens einem zusätzlichen kühlenden Mittel umfasst.

10. Persönliche Sexualgleitmittelzusammensetzung gemäß Anspruch 9, umfassend einen Träger umfassend Wasser; ein verdickendes Polymer, das Hydroxyethylcellulose ist; ein Feuchthaltemittel umfassend Propylenglycol; einen Solubilisator, der PEG-40-hydriertes Rizinusöl ist; einen pH-Wert-Regulator und ein Konservierungsmittel.

11. Persönliche Sexualgleitmittelzusammensetzung gemäß einem der vorstehenden Ansprüche, die als Gel formuliert ist.

12. Verfahren zum Herstellen einer persönlichen Sexualgleitmittelzusammensetzung, umfassend Bereitstellen eines Trägers; eines verdickenden Polymers; eines Vasodilators; eines kühlenden Mittels, das ein cyclisches Carboxamid ist, das gegebenenfalls N-Ethyl-p-menthan-3-carboxamid ist; und gegebenenfalls eines zweiten kühlenden Mittels, und Mischen der Inhaltsstoffe, um die Zusammensetzung zu bilden, wobei die Zusammensetzung frei von Menthol ist.

13. Verfahren gemäß Anspruch 12, wobei das zweite kühlende Mittel (-)-Isopulegol ist.

14. Verfahren gemäß Anspruch 12 oder 13, umfassend Bereitstellen eines Gefäßes; Einfüllen eines Trägers; Zugeben eines Vasodilators und gegebenenfalls eines Konservierungsmittels; Vormischen eines verdickenden Polymers und eines Feuchthaltemittels, um eine erste Vormischung zu erhalten; Zugeben der ersten Vormischung zu dem Gefäß; gesondertes Vormischen eines kühlenden Mittels, das ein cyclisches Carboxamid ist, und eines zweiten kühlenden Mittels und gegebenenfalls eines Stabilisators, um eine zweite Vormischung zu bilden; Zugeben der zweiten Vormischung zu dem Gefäß; Mischen; und gegebenenfalls Einstellen des pH-Werts durch Zugeben von einem oder mehreren pH-Wert-Regulatoren.

## Revendications

1. Composition personnelle de lubrifiant sexuel, comprenant un vasodilatateur et au moins un agent rafraîchissant qui est un carboxamide cyclique, **caractérisée en ce que** la composition est dépourvue de menthol.

2. Composition personnelle de lubrifiant sexuel selon la revendication 1, **caractérisée en ce que** le carboxamide cyclique est le N-éthyl-p-menthane-3-carboxamide.

3. Composition personnelle de lubrifiant sexuel selon la revendication 1 ou 2, comprenant en outre un deuxième agent rafraîchissant, **caractérisée en ce que** le deuxième agent rafraîchissant est le (-)isopulégol, le lactate de menthyle, le glycérol acétal de menthone ou le menthoxypropanediol.

4. Composition personnelle de lubrifiant sexuel selon la revendication 3, **caractérisée en ce que** le deuxième agent rafraîchissant est le (-)isopulégol.

5. Composition personnelle de lubrifiant sexuel selon l'une quelconque des revendications 1 à 4, comprenant en outre un véhicule ; un polymère épaississant ; et éventuellement un agent solubilisant.

6. Composition personnelle de lubrifiant sexuel selon la revendication 5, comprenant un deuxième agent rafraîchissant qui est le (-)isopulégol et un agent solubilisant qui favorise la solubilisation du (-) isopulégol .

7. Composition personnelle de lubrifiant sexuel selon la revendication 6, **caractérisée en ce que** l'agent solubilisant est choisi parmi les huiles de ricin hydrogénées PEG et les polysorbates, éventuellement où l'agent solubilisant est une huile de ricin hydrogénée PEG-40.

8. Composition personnelle de lubrifiant sexuel selon l'une quelconque des revendications précédentes, **caractérisée en ce que** le vasodilatateur est choisi parmi la L-arginine et le trinitrate de glycérol.

9. Composition personnelle de lubrifiant sexuel selon la revendication 5, **caractérisée en ce que** le vasodilatateur comprend de la L-arginine ; le carboxamide cyclique est le N-éthyl-p-menthane-3-carboxamide ; le deuxième agent rafraîchissant est le (-)isopulégol ; laquelle composition comprenant éventuellement un ou plusieurs autres ingrédients choisis parmi au moins un humectant ; au moins un conservateur ; au moins un agent d'ajustement du pH ; au moins un agent solubilisant ; au moins un améliorateur de pénétration ; et au moins un agent rafraîchissant supplémentaire.

10. Composition personnelle de lubrifiant sexuel selon la revendication 9, comprenant un véhicule comprenant de l'eau ; un polymère épaississant qui est l'hydroxyéthylcellulose ; un humectant comprenant du propylène glycol ; un agent solubilisant est une huile de ricin hydrogénée PEG-40 ; un agent d'ajustement du pH ; et un conservateur.

11. Composition personnelle de lubrifiant sexuel selon l'une quelconque des revendications précédentes, qui est formulée sous forme de gel.

12. Méthode de préparation d'une composition personnelle de lubrifiant sexuel, comprenant la fourniture d'un véhicule ; d'un polymère épaississant ; d'un vasodilatateur ; d'un agent rafraîchissant qui est un carboxamide cyclique étant éventuellement le N-éthyl-p-menthane-3-carboxamide ; et éventuellement d'un deuxième agent rafraîchissant ; et le mélange des ingrédients pour former la composition, **caractérisée en ce que** la composition est dépourvue de menthol.

13. Méthode selon la revendication 12, **caractérisée en ce que** le deuxième agent rafraîchissant est le (-)isopulégol.

14. Méthode selon la revendication 12 ou 13, comprenant la fourniture d'un récipient ; le chargement d'un véhicule ; l'addition d'un vasodilatateur et éventuellement d'un conservateur ; le prémélange d'un polymère épaississant et d'un humectant afin de former un premier prémélange ; l'addition du premier prémélange au récipient ; le prémélange séparé d'un agent rafraîchissant qui est un carboxamide cyclique et d'un deuxième agent rafraîchissant et éventuellement d'un stabilisant afin de former un deuxième prémélange ; l'addition du deuxième prémélange au récipient ; le mélange ; et éventuellement l'ajustement du pH par l'addition d'un ou plusieurs agents d'ajustement du pH.
